# EUROPÄISCHE PATENTSCHRIFT

(11) **EP 1 928 361 B1**
(45) Veröffentlichungstag und Bekanntmachung des Hinweises auf die Patenterteilung: **25.10.2017**
(21) Anmeldenummer: 06793559.3
(22) Anmeldetag: 15.09.2006
(51) Int. Cl.: A61F 2/40

(54) **EINSATZ UND SCHALE EINER GELENKKUGELAUFNAHME**
INSERT AND SHELL OF A JOINT BALL RECEPTACLE
PLATEAU ET COQUILLE D'UN DISPOSITIF DE RECEPTION D'UNE ROTULE

(30) Priorität: 16.09.2005 EP 05020296; 01.12.2005 CH 19072005
(43) Veröffentlichungstag der Anmeldung: 11.06.2008
(73) Patentinhaber: Zimmer GmbH, 8404 Winterthur (CH)
(72) Erfinder: FORRER, Michael, CH-8472 Seuzach (CH); GRONAU, Nicole, CH-8400 Winterthur (CH); BOSS, Stefan, CH-8967 Widen (CH); BAUM, Ines, 79283 Bollschweil (DE)
(74) Vertreter: Manitz Finsterwald Patentanwälte PartmbB
(86) Internationale Anmeldenummer: PCT/EP2006/066410
(87) Internationale Veröffentlichungsnummer: WO 2007/031575

(56) Entgegenhaltungen:
- EP-A- 1 472 999
- FR-A- 2 825 263
- GB-A- 2 405 346
- US-A- 5 080 673
- US-B1- 6 679 916

## Beschreibung

Die Erfindung bezieht sich auf einen Einsatz einer Gelenkkugelaufnahme einer Schultergelenkprothese gemäß dem Oberbegriff des Anspruchs 1. Weiterhin bezieht sich die Erfindung auf eine Schale einer Gelenkkugelaufnahme einer Schultergelenkprothese zur Verwendung mit einem solchen Einsatz, eine aus einem solchen Einsatz und einer solchen Schale bestehende Gelenkkugelaufnahme einer Schultergelenkprothese und auch auf eine komplette Schultergelenkprothese.

Bei Schultergelenken hängt die Auswahl des passenden Implantats und die Art und Weise der Implantation entscheidend davon ab, in welchem Zustand sich die beteiligten Knochen befinden, wobei auch der Zustand der Muskeln, insbesondere der Rotatorenmanschette, eine Rolle spielt. Beim Schultergelenk kommt es insbesondere auf den Zustand des Schulterblatts und dabei vor allem der Gelenkpfanne, dem Glenoid an, die beim gesunden Gelenk mit dem Kopf des Oberarms, dem Humerus, zusammenwirkt. Die Notwendigkeit für einen teilweisen oder vollständigen Ersatz des Schultergelenks kann aus unterschiedlichen Gründen bestehen. Typische Ursachen sind beispielsweise fortgeschrittener Verschleiß der Gelenkflächen oder Frakturen z.B. aufgrund eines Unfalls. Je nach Art und Ausmaß der Schädigung kann auch eine so genannte inverse Prothesenkonfiguration angezeigt sein, bei welcher die künstliche Gelenkkugel und die künstliche Gelenkaufnahme bezüglich ihrer Positionen in einem natürlichen Gelenk vertauscht sind.

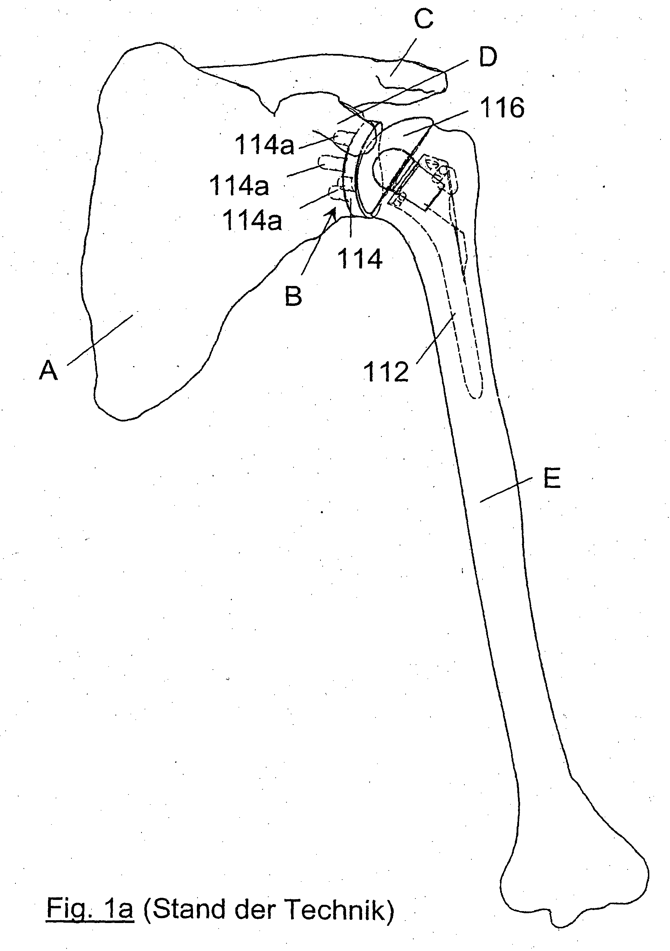

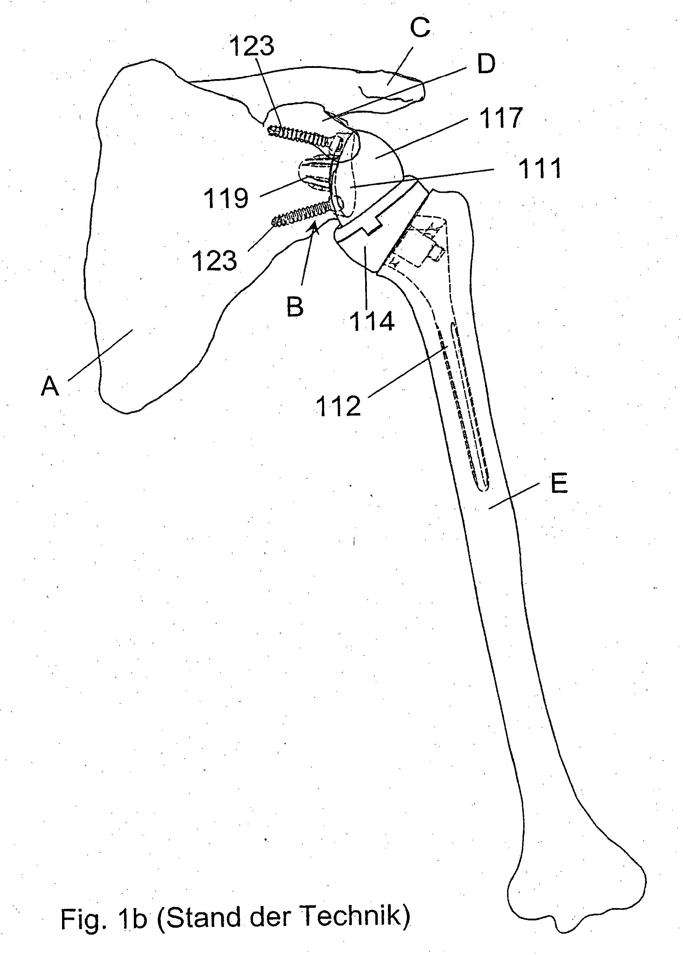

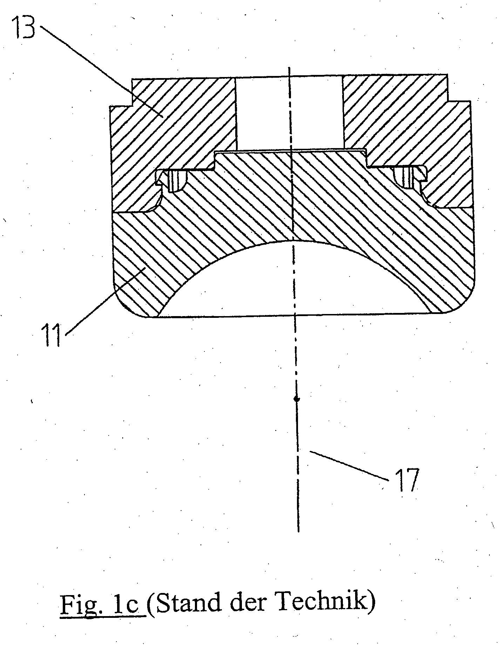

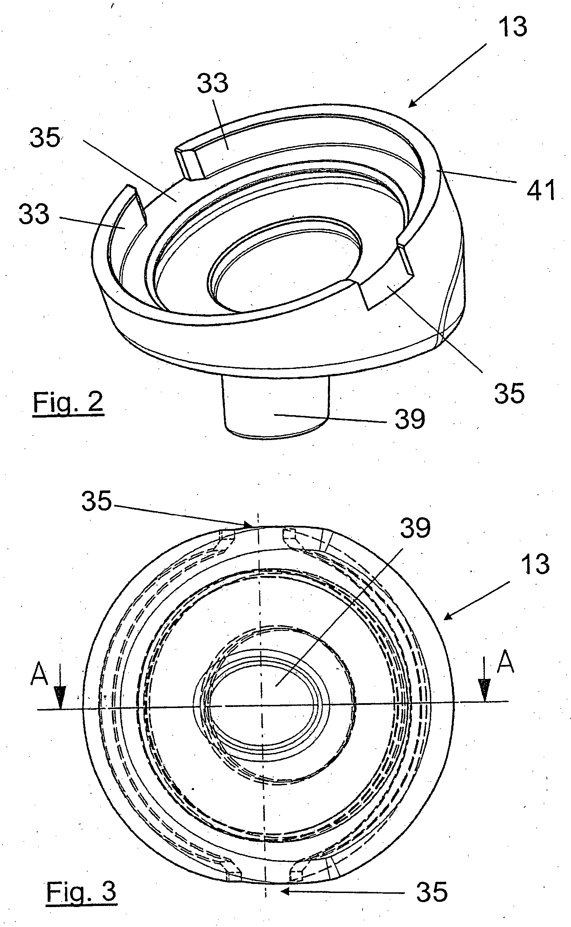

Bevor anhand der Fig. 2 bis 15 mögliche Ausführungsformen der hier vorgeschlagenen Gelenkkugelaufnahme vorgestellt werden, soll zunächst anhand der Fig. 1a, 1b und 1c ein einleitender Überblick gegeben werden.

Die knöcherne Struktur des Schultergelenks besteht aus dem Kopf des Oberarmknochens E und der Gelenkpfanne B des Schulterblatts A. Für die Funktion des Schultergelenks von Bedeutung sind außerdem zwei Knochenvorsprünge des Schulterblatts A, nämlich das Acromion C und das Coracoid D. Diese Knochenvorsprünge bilden zusammen mit einem nicht dargestellten sie verbindenden Band das so genannte Schulterdach, das eine gewölbeartige Form aufweist und ein Abwandern des Oberarmkopfes aus der Gelenkpfanne nach oben verhindert. Fig. 1a zeigt eine so genannte anatomische Konfiguration, bei welcher die Prothese das Schultergelenk in seinem natürlichen Aufbau nachbildet, d. h. der Humerus E ist mit einem künstlichen Gelenkkopf 116 und das Schulterblatt A mit einer künstlichen Gelenkkugeläufnahme oder Gelenkpfanne 114 versehen. Die Verankerung der Gelenkpfanne 114 am Schulterblatt A, genauer gesagt am entsprechend vorbereiteten Glenoid B, erfolgt im dargestellten Beispiel über Schrauben 114a. Die Verankerung des künstlichen Gelenkkopfes 116 am Humerus E erfolgt mittels eines Schaftes 112, der verschiedenartig ausgestaltet sein kann.

Fig. 1b zeigt eine so genannte inverse Konfiguration, bei welcher der künstliche Gelenkkopf und die künstliche Gelenkpfanne 114' bezüglich ihrer Positionen im natürlichen Schultergelenk vertauscht sind. Der Gelenkkopf ist hier von einem künstlichen Gelenkteil gebildet, das eine Basisplattform 111 und eine mit der Plattform 111 verbundene Kugelkomponente 117 umfasst. Die Verankerung der Plattform 111 am Schulterblatt A, genauer gesagt am entsprechend vorbereiteten Glenoid B, erfolgt im dargestellten Beispiel über einen lediglich schematisch angedeuteten Zapfen 119 der Plattform 111 sowie mittels Schrauben 123, für die in der Plattform 111 entsprechende Schraubenaufnahmen vorgesehen sind. Während die Plattform 111 hier also die künstliche Kugelkomponente 117 trägt, ist am Humerus E mittels eines Schaftes 112 die künstliche Gelenkpfanne 114' verankert, welche die Gelenkkugelaufnahme bildet.

Es ist im Stand der Technik bekannt, Gelenkkugelaufnahmen mit einer im Knochen zu verankernden Schale und einem darin zu befestigenden Einsatz auszuführen. Es ist bekannt, die Schale aus Metall und den Einsatz, welcher beispielsweise eine Vertiefung zur Aufnahme einer Gelenkkugel aufweist, aus Kunststoff auszuführen. Zur Verankerung eines Einsatzes in einer Schale ist es beispielsweise gemäß Fig. 1c bekannt, die Innenkontur der Schale 13 mit einer Hinterschneidung zu versehen, und den Einsatz 11 derart mit einem umlaufend ausgebildeten sich radial nach außen erstreckenden Kragen zu versehen, dass sich ein in Umfangsrichtung verlaufendes, sich radial nach außen erstreckendes, im Querschnitt hakenförmiges Hinterschneidungselement ausbildet. Dieses Element kann durch elastische Verformung mit der Hinterschneidung der Schale 13 in Eingriff gebracht werden, und zwar derart, dass der Einsatz 11 in der Schale 13 verankert ist, wie es Fig. 1c zeigt.

Bei einer Gelenkkugelaufnahme der hier angegebenen Art ist der Kragen des Schnappmechanismus an wenigsten einer Stelle seines Umfangs unterbrochen. Dies bewirkt, dass der Kragen beim Einführen des Einsatzes in eine Schale stärker verformt werden kann, was sich dann in einer größeren Rückstellbewegung der Hinterschneidung nach dem vollständigen Einführen des Einsatzes niederschlägt. Durch diese größere Rückstellbewegung kann die Hinterschneidung das entsprechende Gegenelement in der Schale in einem vergleichsweise großen Maße bzw. mit einer relativ großen "Tiefe" hintergreifen. So lässt sich letztlich ein guter und großflächiger Formschluss zwischen Einsatz und Schale bzw. zwischen Hinterschneidung des Einsatzes und Gegenelement der Schale erreichen. Weiterhin ist der Einsatz mit einem zylindrischen Führungsbereich versehen. Der Einsatz kann mit einer Schale nach einem der auf eine Schale gerichteten Ansprüche kombiniert werden. Auf diese Weise erhält man bei mit der Schale gekoppeltem Einsatz eine weitgehend vollflächige Anlage der Außenseite des zylindrischen Führungsbereichs des Einsatzes an der Innenseite des zylindrischen Innenführungsbereichs der Schale. Der zylindrische Führungsbereich übernimmt eine Führungs- und/oder Zentrierfunktion des Einsatzes in der Schale. Insbesondere wird die mit dem Schnappmechanismus erzielbare Funktion des Koppelns von Einsatz und Schale von der Funktion des Führens und/oder Zentrierens des Einsatzes getrennt. Aus GB 2 405 346 ist ein Einsatz zur Gelenkkugelaufnahme einer Schulterprothese bekanntgeworden, welche an einem axialen Ende einen Schnappmechanismus und weiterhin einen zylindrischen Führungsbereich aufweist.

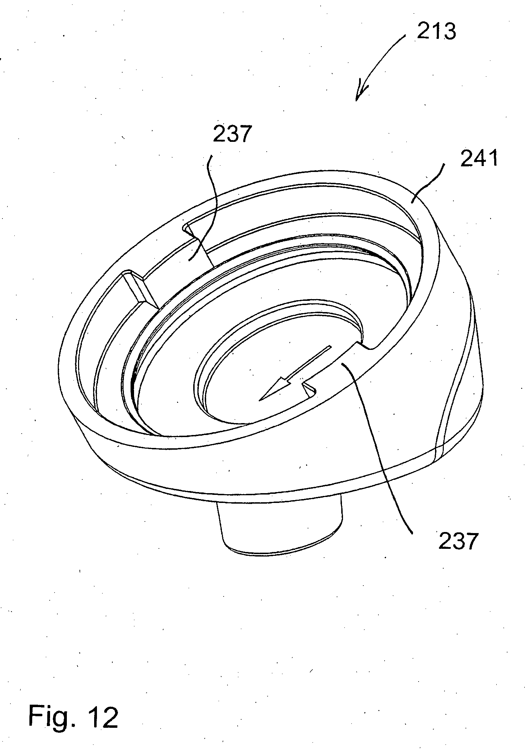

Ein Ausführungsbeispiel ist in den Figuren 2 bis 11 dargestellt. Ein weiteres Ausführungsbeispiel ist in den Figuren 12 bis 15 dargestellt.

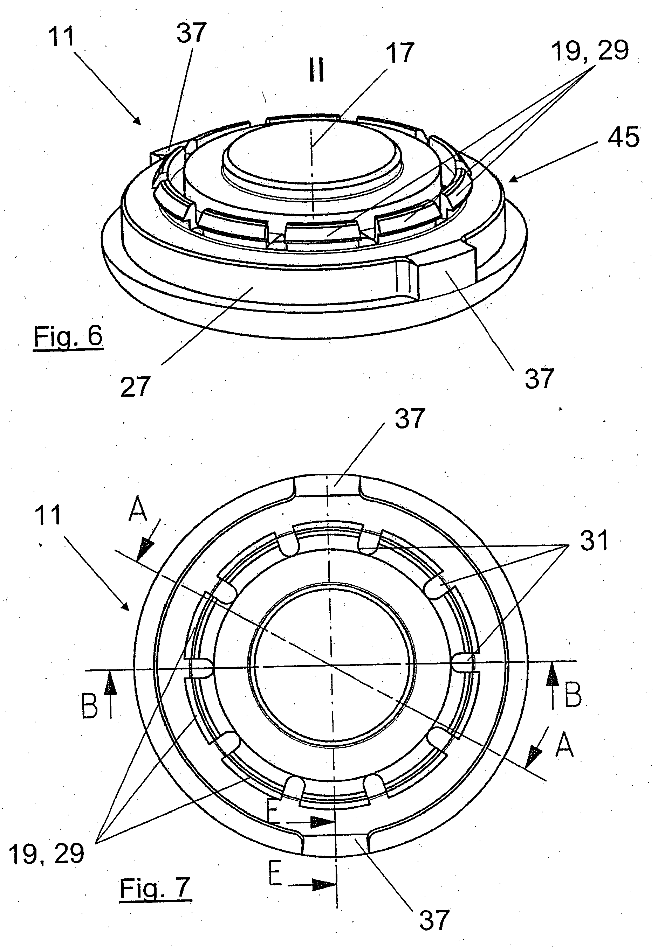

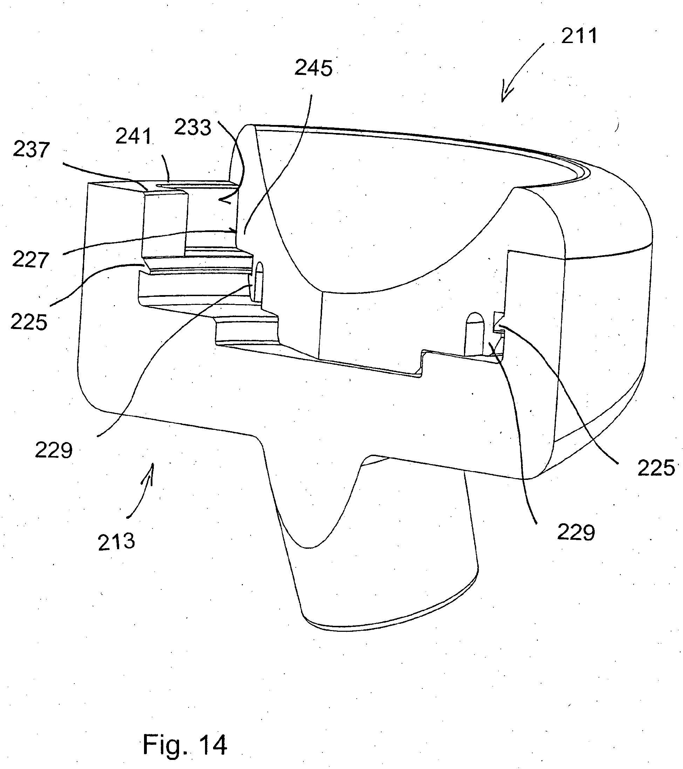

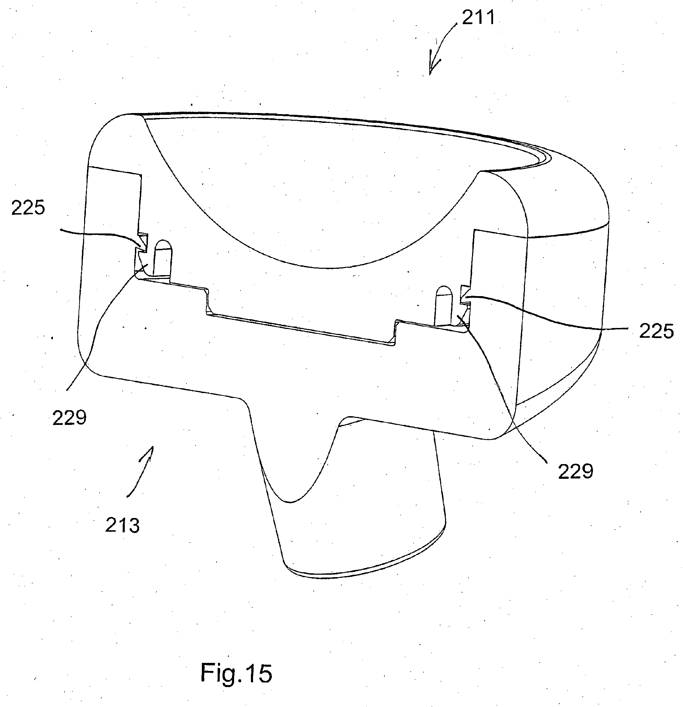

Fig. 2 bis 5, 12 zeigen eine Schale, Fig. 6 bis 11, 13 einen Einsatz, und Fig. 14, 15 eine Gelenkkugelaufnahme der in den Ansprüchen gekennzeichneten Art.

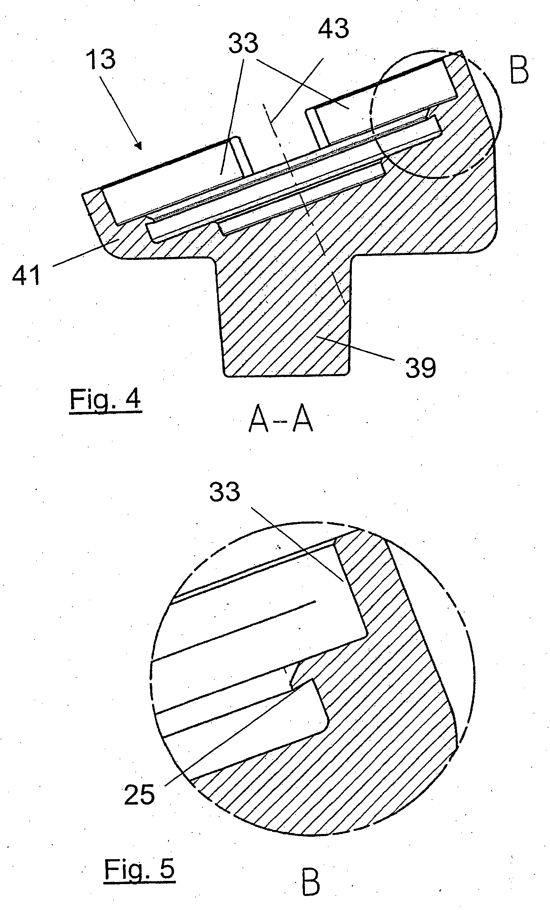

Die Schale 13 gemäß Fig. 2 bis 5 weist einen sich konisch verjüngenden Zapfen 39 auf, mit dem die Schale 13 an einem nicht dargestellten Humerusschaft oder am Schulterblatt, gegebenenfalls mit einer dazwischen angeordneten Plattform, verankert werden kann. Die äußere Querschnittsform des Konuszapfens 39 ist elliptisch. Auf nähere Einzelheiten zu der Schalenverankerung soll an dieser Stelle nicht eingegangen werden, da diese Verankerung nicht Gegenstand der Erfindung ist. Die Schale 13 weist an ihrer zur Aufnahme des nachstehend an Hand der Fig. 6 bis 11 beschriebenen Einsatzes 11 vorgesehenen Seite einen zylindrischen Abschnitt 41 in Form einer Umfangswand auf, dessen Mittelachse 43 gemäss Fig. 4 gegenüber einer Mittelachse des Konuszapfens 39 geneigt ist. Der Zylinderabschnitt 41 ist an zwei einander diametral gegenüberliegenden Stellen unterbrochen. Die hierdurch entstehenden Aussparungen 35 dienen zur Aufnahme von entsprechenden Vorsprüngen 37 des Einsatzes 11 gemäss Fig. 6. Hierdurch wird eine Verdrehsicherung des mit der Schale 13 gekoppelten Einsatzes 11 erzielt. Die Innenseite der zylindrischen Umfangswand 41 dient als zylindrischer Innenführungsbereich 33 für einen entsprechenden zylindrischen Führungsbereich 27 des Einsatzes 11 gemäss Fig. 6. Am "Grund" der Aufnahme ist eine weitere zylindrische Vertiefung zu erkennen (ohne Bezugszeichen), welche, wie in den Figuren 14 und 15 zu erkennen ist, zur Aufnahme einer zweiten Führung des Einsatzes vorgesehen ist.

In Fig. 3, die eine Ansicht der Schale 13 von "unten" gemäss Figur 2, das heisst von ihrer Verankerungsseite her, zeigt, ist insbesondere die elliptische äußere Querschnittsform des Konuszapfens 39 zu erkennen.

Der Fig. 4, die einen Schnitt entlang der Linie "A-A" von Fig. 3 zeigt, und der Fig. 5, die das Detail "B" von Fig. 4 zeigt, ist insbesondere zu entnehmen, dass die Aufnahmeseite der Schale 13 für den Einsatz 11 im Wesentlichen von zwei zylindrischen Bereichen mit unterschiedlichem Innendurchmesser gebildet ist. Der Bereich mit größerem Innendurchmesser ist durch den bereits erwähnten zylindrischen Innenführungsbereich 33 begrenzt und von dem Bereich mit kleinerem Innendurchmesser durch einen radial nach innen vorstehenden, umlaufenden Vorsprung getrennt, der eine Hinterschneidung bereitstellt und als Gegenelement 25 für einen nachstehend näher beschriebenen Schnappmechanismus 19 des Einsatzes 11 dient, siehe Figur 6.

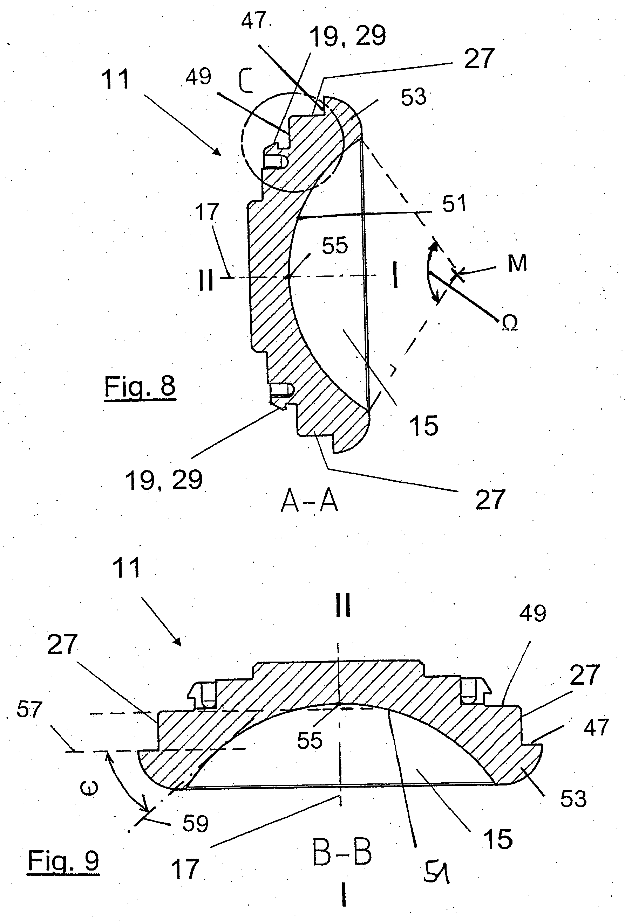

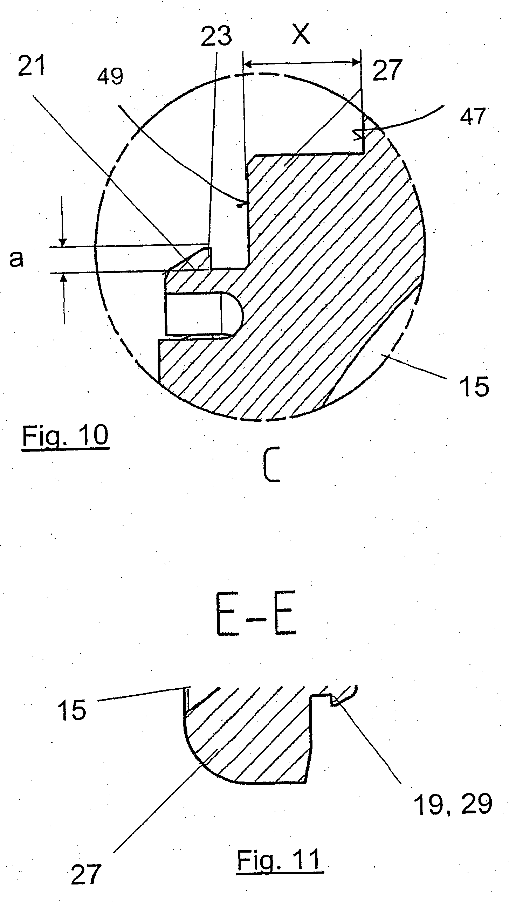

Der in den Fig. 6 bis 11 dargestellte Einsatz 11 ist mit einem umlaufenden Zylinderabschnitt 45 versehen, der an seiner Außenseite einen zylindrischen Führungsbereich 27 aufweist, der sich parallel zur Mitten-achse 17 des Einsatzes 11 erstreckt, die mit der Mittelachse des Zylinderabschnitts 45 zusammenfällt. Der Zylinderabschnitt 45 des Einsatzes 11 ist entsprechend dem Aufnahmebereich mit größerem Innendurchmesser der Schale 13 dimensioniert, so dass im zusammengesetzten Zustand der zylindrische Führungsbereich 27 des Einsatzes 11 und der zylindrische Innenführungsbereich 33 der Schale 13 einander gegenüberliegen und zusammenwirken, wie es im Einleitungsteil beschrieben ist. Die Abmessungen sind dabei derart gewählt, dass der Einsatz 11 mit geringer Toleranz in die Schale 13 eingesteckt wird. Insbesondere sind die Durchmessertoleranzen hier so bemessen, dass der zylindrische Führungsbereich des Einsatzes ohne übermässigen Kraftaufwand in die Schale einbringbar ist, und andererseits eine gute Zentrierung und Führung gewährleistet ist. In einem möglichen Ausführungsbeispiel kann der Durchmesser des zylindrischen Innenführungsbereiches 33 der Schale 13 gegenüber dem Durchmesser des zylindrischen Führungsbereiches 27 des Einsatzes 11 ein Übermaß von maximal 1/10 mm oder maximal 1 %, zum Beispiel weniger als 0,4 %, des Durchmessers des zylindrischen Führungsbereiches 27 des Einsatzes 11 aufweisen. Wie insbesondere in Fig. 10 gezeigt ist, schließt der zylindrische Führungsbereich 27 mit seinem einen axialen Ende über eine erste, senkrecht zu der Achse des zylindrischen Führungsbereichs 27 verlaufende ringförmige Fläche 47 an einen im Bereich der ersten Seite I ausgebildeten Abschnitt 53 des Einsatzes 11 an, siehe Figuren 8 und 9, an welchem der Einsatz 11 seinen maximalen Außendurchmesser aufweist, wobei sich die erste ringförmige Fläche 47 von dem zylindrischen Führungsbereich 27 radial nach außen erstreckt, so dass der Außendurchmesser des zylindrischen Führungsbereichs 27 dem Innendurchmesser der ersten ringförmigen Fläche 47 entspricht. Der Abschnitt 53 des Einsatzes 11 ist im mit der Schale 13 gekoppelten Zustand außerhalb der Schale 13 angeordnet, so dass der Außendurchmesser des zylindrischen Führungsbereichs 27 im mit der Schale 13 gekoppelten Zustand den größten innerhalb der Schale 13 liegenden Durchmesser des Einsatzes 11 bildet. Mit seinem anderen Ende schließt der zylindrische Führungsbereich 27 über eine zweite, senkrecht zu der Achse des zylindrischen Führungsbereichs 27 verlaufende ringförmige Fläche 49 an den nachstehend näher beschriebenen Schnappmechanismus 19 des Einsatzes 11 an, wobei sich die zweite ringförmige Fläche 49 von dem zylindrischen Führungsbereich 27 weg radial nach innen erstreckt, so dass der Außendurchmesser des zylindrischen Führungsbereichs 27 dem Außendurchmesser der zweiten ringförmigen Fläche 49 entspricht. Eine der beiden ringförmigen Flächen 47 oder 49 wirkt dabei als axialer Anschlag für eine entsprechend ausgebildete Gegenfläche der Schale 13. Mit dem Gegenelement 25 der Schale 13 wirken eine Mehrzahl von Schnappelementen 29 des Einsatzes 11 zusammen, die jeweils als hakenförmiges Umfangssegment ausgebildet und radial innerhalb des zylindrischen Führungsbereiches 27 angeordnet sind. Die Schnappelemente 29 sind in Umfangsrichtung gleichmäßig verteilt angeordnet, wobei jeweils zwischen zwei benachbarten Schnappelementen 29 eine Unterbrechung 31 vorhanden ist. In einer möglichen Ausführungsform können die Unterbrechungen 31 jeweils eine Breite von wenigstens 1 mm aufweisen. Die Unterbrechungen 31 erstrecken sich in axialer Richtung jeweils von der senkrecht zur Mittenachse 17 verlaufenden Stirnfläche des Zylinderabschnitts 45 bis zum freien axialen Ende der Schnappelemente 29. Die Gesamtheit der Schnappelemente 29 bildet einen Schnappmechanismus 19 in Form eines umlaufenden unterbrochenen Kragens 21 mit einer Hinterschneidung 23, siehe Fig. 10, wobei der maximale Außendurchmesser des Schnappmechanismus 19 kleiner ist als der Außendurchmesser des zylindrischen Führungsbereichs 27, welcher näher an der ersten Seite I angeordnet ist, als der Schnappmechanismus 19. Die bezüglich des Zylinderabschnitts 45 radial vorstehenden, einander diametral gegenüberliegenden Vorsprünge 37 des Einsatzes 11 wirken mit den Aussparungen 35 der Schale 13 zusammen, um - wie vorstehend bereits erwähnt - den Einsatz 11 im gekoppelten Zustand verdrehsicher in der Schale 13 zu halten. Der Schnappmechanismus 19 des Einsatzes 11 ist auf einer im zusammengesetzten Zustand der Schale 13 zugewandten zweiten Seite II des Einsatzes 11 ausgebildet, wohingegen - wie insbesondere die Fig. 8 und 9 zeigen - auf der gegenüberliegenden ersten Seite I des Einsatzes 11 eine Vertiefung 15 ausgebildet ist, welche die eigentliche Aufnahme für die hier nicht dargestellte Gelenkkugel der Prothese bildet. Weiterhin ist am axialen Ende den zweiten Seite II ein zweiter Führungsbereich (ohne Bezugszeichen) angeordnet, welchen mit einer entsprechenden Aufnahme der Schale zusammenwirken vermag.

Die nachfolgenden Ausführungen erfolgen unter Bezug auf die Figuren 8 und 9. Die Vertiefung 15 ist kugelabschnittförmig ausgebildet und weist einen sphärischen Oberflächenbereich 51 auf, welcher sich bezüglich eines Mittelpunkts M des sphärischen Oberflächenbereichs 51 über einen Winkelbereich Ω von beispielsweise 110°, d.h. über einen Halbwinkelbereich Ω/2 von beispielsweise 55°, erstreckt. Der Winkel Ω überschreitet bei Schultergelenkslagerschalen insbesondere einen Winkel von 120° nicht. In anderen Ausführungsformen wird Ω auf Maximalwerte von 110° oder gar 100° oder 90° begrenzt, insbesondere um den Beweglichkeitsbereich des Schultergelenkes nicht einzuschränken und weil ein Sitz und die Zentrierung der Gelenkkugel bei Schultergelenken ohnehin in einem hohen Ausmasse durch den Band- und Muskulaturapparat erfolgt; dies beispielsweise im Vergleich zu Hüftgelenken, wo ein vergleichbarer Umfassungswinkel der Lagerschale in die Grössenordnung von 180° kommt. Die Vertiefung 15 weist weiterhin einen Pol 55 auf, der auf dem Schnittpunkt der Mittenachse 17 mit dem sphärischen Oberflächenbereich 51 liegt, wobei die axiale Position des der zweiten Seite II zugewandten axialen Endes des zylindrischen Führungsbereichs 27 geringfügig, beispielsweise um 0,5 mm, zu der axialen Position des Pols 55 in Richtung der ersten Seite I versetzt ist. Grundsätzlich ist es auch möglich, dass die axiale Position des der zweiten Seite II zugewandten axialen Endes des zylindrischen Führungsbereichs 27 geringfügig in Richtung der zweiten Seite II versetzt ist. Das der zweiten Seite II zugewandte axiale Ende des zylindrischen Führungsbereichs 27 kann also - von der ersten Seite I aus betrachtet - auch "tiefer" angeordnet sein, als der Pol 55 der Vertiefung 15. Fig. 9 zeigt ferner einen Steigungswinkel ω, der zwischen einer Senkrechten 57 zu der Mittenachse 17 und einer Tangente 59 an dem sphärischen Oberflächenbereich 51 ausgebildet ist. Die Tangente 59 ist an einer axialen Position des sphärischen Oberflächenbereichs 51 angelegt, die der axialen Position des der ersten Seite I zugewandten Endes des zylindrischen Führungsbereichs 27 entspricht. Der Steigungswinkel ω beträgt beispielsweise in der dargestellten Ausführungsform weniger als 45°, und kann bis zum Winkel Ω/2 gehen. Andere Steigungswinkel, die an axialen Positionen an dem sphärischen Oberflächenbereich 51 vorliegen, die den axialen Positionen anderer Bereiche des zylindrischen Führungsbereichs 27 entsprechen, liegen folglich im Bereich zwischen 0° und dem Steigungswinkel ω, welcher der axialen Position des der ersten Seite I zugewandten Endes des zylindrischen Führungsbereichs 27 entspricht. Fig. 10 ist des Weiteren zu entnehmen, dass die axiale Erstreckung x des Zylinderabschnitts 45 und damit des zylindrischen Führungsbereiches 27 des Einsatzes 11 ein Vielfaches der in radialer Richtung bezüglich der Mittenachse 17 gemessenen Tiefe a der Hinterschneidung 23 des von den Schnappelementen 29 gebildeten Kragens 21 beträgt. In einer möglichen Ausführungsform beträgt die Tiefe a der Hinterschneidung wenigstens 1 mm, beispielsweise mehr als 1,3 mm. Bezogen auf den Außendurchmesser des Schnappmechanismus 19 kann die Tiefe a der Hinterschneidung 23 in einer möglichen Ausführungsform wenigstens 3 %, in einer Ausführungsform wenigstens 3,5 %, dieses Außendurchmessers betragen. Die axiale Erstreckung x des zylindrischen Führungsbereichs 27 parallel zur Mittenachse 17 des Einsatzes 11 kann wenigstens 3 mm, insbesondere wenigstens 3,5 mm, betragen. Bezogen auf den Außendurchmesser des zylindrischen Führungsbereiches 27 kann dessen axiale Erstreckung x parallel zur Mittenachse 17 wenigstens 10 % dieses Außendurchmessers betragen. Was die axiale Erstreckung des zylindrischen Innenführungsbereichs 33 der Schale 13 anbetrifft, so ist diese zumindest und in einer Ausführungsform im Wesentlichen genauso groß wie die axiale Erstreckung x des zylindrischen Führungsbereichs 27 des aufzunehmenden Einsatzes 11.

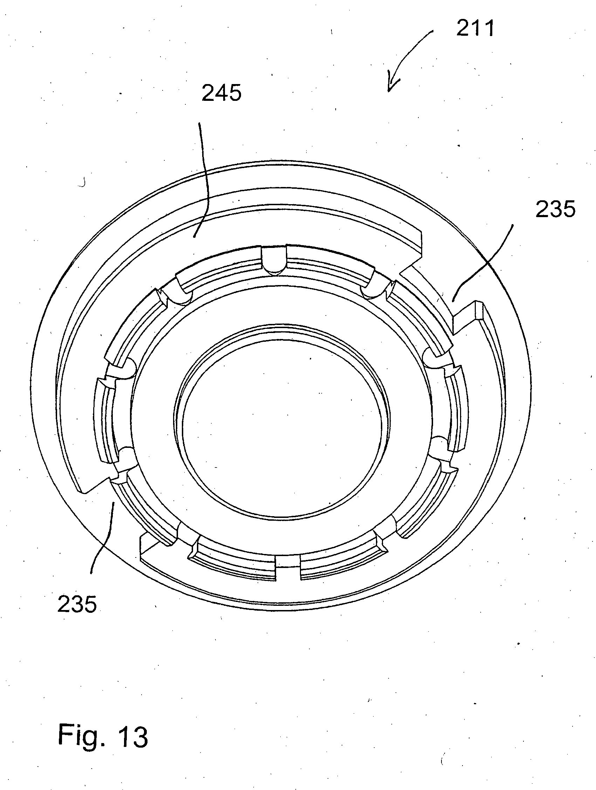

In einem weiteren Ausführungsbeispiel gemäß den Figuren 12 und 13 ist eine Verdrehsicherung vorgesehen, deren Komponenten, Aussparung und Vorsprung, gegenüber der im Zusammenhang mit dem Ausführungsbeispiel gemäß den Figuren 2 bis 11 erläuterten Verdrehsicherung hinsichtlich ihrer Anordnung an Schale und Einsatz gerade vertauscht sind. Die in Fig. 12 gezeigte Schale 213 umfasst einen Zylinderabschnitt 241, der an zwei einander diametral gegenüberliegenden Stellen jeweils einen radial nach innen vorstehenden Vorsprung 237 aufweist. Die Vorsprünge 237 werden im zusammengesetzten Zustand von entsprechenden Aussparungen 235 des Einsatzes 211 aufgenommen, welche in einem Zylinderabschnitt 245 des Einsatzes 211 an zwei einander diametral gegenüberliegenden Stellen vorgesehen sind. Ansonsten entspricht das Ausführungsbeispiel gemäß den Figuren 12 und 13 dem Ausführungsbeispiel gemäß den Figuren 2 bis 11, wobei nachstehend zu den Figuren 14 und 15 bereits vorstehend erläuterte Aspekte nochmals aufgegriffen bzw. wiederholt werden.

In den Figuren 14 und 15 ist ein Längsschnitt durch eine aus der Schale 213 gemäß Figur 12 und dem Einsatz 211 gemäß Fig. 13 zusammengesetzte Gelenkkugelaufnahme entlang der Mittenachse des Einsatzes 211 bzw. der Mittelachse des Zylinderabschnitts 241 der Schale 213 gezeigt. Der Zylinderabschnitt 245 des Einsatzes 211 weist an seiner Außenseite einen zylindrischen Führungsbereich 227 auf, der mit einem zylindrischen Innenführungsbereich 233 des Zylinderabschnitts 241 der Schale 233 derart zusammenwirkt, dass eine vollflächige Anlage der Zylinderabschnitte 241 und 245 aneinander realisiert ist. Der Einsatz 211 weist weiterhin mehrere hinterschnittene, elastisch verformbare Schnappelemente 229 auf, die hinter einen umlaufenden Vorsprung 225 der Schale 213 eingreifen, so dass zwischen dem Einsatz 211 und der Schale 213 eine Schnappverbindung ausgebildet ist.

Der in den Figuren dargestellte Einsatz 11 ist in einer Ausführungsform aus Kunststoff, insbesondere aus Polyethylen, gefertigt. Die mindeste Materialstärke des tragenden Bereiches, benachbart zu der zur Aufnahme der Gelenkkugel vorgesehenen Vertiefung 15, beträgt beispielsweise nicht weniger als 3 mm, und liegt in spezifischen Ausführungsformen bei 3,4 mm bis 4,0 mm, spezifischer um 3,5 mm, allenfalls in einem Toleranzbereich von ± 0,5 mm. Die Materialstärke wird dabei in radialer Richtung der kugel- oder kalottenförmigen Vertiefung 15 zur Aufnahme der Gelenkkugel, das heißt in Richtung der oberflächennormalen Krafteinleitung, gemessen.

Die hier beschriebene Gelenkkugelaufnahme 11, 13 kann konventionell - in einer auch als "anatomisch" bezeichneten Konfiguration - an der Scapula befestigt sein. Sehr gut eignen sich die beschriebenen Komponenten auch zur Ausbildung einer Schultergelenkprothese, bei der die Gelenkkugelaufnahme 11, 13 - in einer auch als "invers" bezeichneten Konfiguration - zur Befestigung am Humerus vorgesehen ist. Dabei ist die Gelenkkugelaufnahme 11, 13 beispielsweise an einem an sich bekannten Schaft befestigt, wie er zur Befestigung in einem langen Knochen Verwendung findet, und welcher sich - je nach Ausführung zementiert oder unzementiert - gut im Humerus verankern lässt.

### Bezugszeichenliste

- 11: Einsatz
- 13: Schale
- 15: Vertiefung
- 17: Mittenachse des Einsatzes
- 19: Schnappmechanismus
- 21: Kragen
- 23: Hinterschneidung des Schnappmechanismus
- 25: umlaufender Vorsprung, Gegenelement der Schale
- 27: zylindrischer Führungsbereich des Einsatzes
- 29: Umfangssegmente, Schnappelemente des Schnappmechanismus
- 31: Unterbrechung
- 33: zylindrischer Innenführungsbereich der Schale
- 35: Aussparung der Schale zur Verdrehsicherung
- 37: Vorsprung des Einsatzes zur Verdrehsicherung
- 39: Konus der Schale
- 41: Zylinderabschnitt der Schale
- 43: Mittelachse des Zylinderabschnitts der Schale
- 45: Zylinderabschnitt des Einsatzes
- 47: ringförmige Fläche
- 49: ringförmige Fläche
- 51: sphärischer Oberflächenbereich
- 53: Abschnitt des Einsatzes
- 55: Pol des sphärischen Oberflächenbereichs
- 57: Senkrechte zur Mittenachse
- 59: Tangente an dem sphärischen Oberflächenbereich

- x: axiale Erstreckung des zylindrischen Führungsbereiches des Einsatzes
- a: Tiefe der Hinterschneidung des Schnappmechanismus
- M: Mittelpunkt des sphärischen Oberflächenbereichs

- Ω: Winkelbereich
- ω: Steigungswinkel

- I: erste Seite des Einsatzes
- II: zweite Seite des Einsatzes

- 111: Basisplattform
- 112: Schaft
- 114, 114': künstliche Gelenkpfanne
- 114a: Schraube
- 116: künstlicher Gelenkkopf
- 117: künstliche Kugelkomponente
- 119: Zapfen
- 123: Schraube

- 211: Einsatz
- 213: Schale
- 225: umlaufender Vorsprung, Gegenelement der Schale
- 227: zylindrischer Führungsbereich des Einsatzes
- 229: Umfangssegmente, Schnappelemente des Schnappmechanismus
- 233: zylindrischer Innenführungsbereich der Schale
- 235: Aussparung des Einsatzes zur Verdrehsicherung
- 237: Vorsprung der Schale zur Verdrehsicherung
- 241: Zylinderabschnitt der Schale
- 245: Zylinderabschnitt des Einsatzes

## Patentansprüche

1. Einsatz einer Gelenkkugelaufnahme einer Schultergelenkprothese, wobei der Einsatz (11) eine erste Seite (I) mit einer Vertiefung (15) zur Aufnahme einer Gelenkkugel aufweist, sowie eine zweite Seite (II), welche zur Aufnahme in einer Schale (13) der Gelenkkugelaufnahme vorgesehen und ausgebildet ist,
wobei im Bereich der zweiten Seite (II) ein sich um eine Mittenachse (17) des Einsatzes (11) erstreckender Schnappmechanismus (19) angeordnet ist, welcher von der zweiten Seite (II) aus gesehen zusammen mit einem Kragen (21) eine sich um die Mittenachse (17) erstreckende Hinterschneidung (23) aufweist, derart, dass der Kragen (21) durch elastische Verformung mit einem entsprechend ausgebildeten Gegenelement (25) der Schale (13) in Hintergriff bringbar ist, und dass der Schnappmechanismus (19) in Umfangsrichtung des Einsatzes (11) an wenigstens einer Stelle (31) unterbrochen ist, und
wobei der Einsatz (11) in dem zur Aufnahme in der Schale - (13) vorgesehenen Bereich einen zylindrischen Führungsbereich (27) am Außenumfang aufweist, dessen Aussendurchmesser grösser ist als der maximale Aussendurchmesser des Schnappmechanismus und welcher vom Schnappmechanismus aus gesehen zur ersten Seite hin angeordnet ist, **dadurch gekennzeichnet, dass** der Einsatz an der zweiten Seite einen zweiten Führungsbereich aufweist, welcher in axialer Richtung des Einsatzes vom Schnappmechanismus aus gesehen zur zweiten Seite hin angeordnet ist.

2. Einsatz gemäß Anspruch 1,
**dadurch gekennzeichnet,**
**dass** die Achse des zylindrischen Führungsbereichs (27) mit der Mittenachse (17) des Einsatzes (11) zusammenfällt.

3. Einsatz gemäß einem der Ansprüche 1 oder 2,
**dadurch gekennzeichnet,**
**dass** der Einsatz (11) im Bereich der ersten Seite (I) einen maximalen Außendurchmesser aufweist, an den sich nach einer stufenartigen Durchmesserverkleinerung der zylindrische Führungsbereich (27) anschließt.

4. Einsatz gemäß einem der vorstehenden Ansprüche,
**dadurch gekennzeichnet,**
**dass** der zylindrische Führungsbereich Mittel (37, 235) zur formschlüsigen Verdrehsicherung des Einsatzes aufweist.

5. Einsatz gemäß einem der vorstehenden Ansprüche,
**dadurch gekennzeichnet,**
**dass** im mit der Schale (13) gekoppelten Zustand der Außendurchmesser des zylindrischen Führungsbereichs (27) den größten innerhalb der Schale (13) liegenden Durchmesser des Einsatzes (11) darstellt.

6. Einsatz gemäß einem der vorstehenden Ansprüche,
**dadurch gekennzeichnet,**
**dass** sich an beiden axialen Enden des zylindrischen Führungsbereichs (27) jeweils eine quer, insbesondere senkrecht, zu der Achse des zylindrischen Führungsbereich (27) verlaufende ringförmige Fläche (47, 49) anschließt, von denen eine als axiale Anschlagsfläche für die Schale (13) ausgebildet ist.

7. Einsatz gemäß einem der vorstehenden Ansprüche,
**dadurch gekennzeichnet,**
**dass** die Vertiefung (15) einen sphärischen Oberflächenbereich (51) aufweist, welcher sich bezüglich eines Mittelpunkts (M) des sphärischen Oberflächenbereichs (51) über einen Winkelbereich (Ω) von weniger als 120° erstreckt.

8. Einsatz gemäß einem der vorstehenden Ansprüche,
**dadurch gekennzeichnet,**
**dass** das der ersten Seite (I) zugewandte axiale Ende des zylindrischen Führungsbereichs (27) eine axiale Position einnimmt, an welcher eine Tangente an einem sphärischen Oberflächenbereich (51) der Vertiefung (15) mit einer Senkrechten zu der Mittenachse (17) einen Steigungswinkel (ω) von weniger als 60°, insbesondere weniger als 45°, einschließt.

9. Einsatz gemäß einem der vorstehenden Ansprüche,
**dadurch gekennzeichnet,**
**dass** der zylindrische Führungsbereich (27) in Richtung der Mittenachse (17) eine axiale Position einnimmt, welche sich im Bereich der axialen Position eines Pols der Vertiefung (15) befindet.

10. Schale einer Gelenkkugelaufnahme einer Schultergelenkprothese zur Aufnahme eines Einsatzes gemäß einem der vorstehenden Ansprüche,
**dadurch gekennzeichnet,**
**dass** die Schale (13) auf ihrer Innenseite einen zylindrischen Innenführungsbereich (33) zur Aufnahme des zylindrischen Führungsbereichs (27) des Einsatzes (11) aufweist, und weiterhin aus Grund der Aufnahmeöffnung einen weiteren Innenführungsbereich aufweist.

11. Schale gemäß Anspruch 10,
**dadurch gekennzeichnet,**
**dass** die axiale Erstreckung des zylindrischen Innenführungsbereichs (33) der Schale (13) zumindest und insbesondere im Wesentlichen genauso groß ist, wie die axiale Erstreckung (x) des zylindrischen Führungsbereichs (27) des aufzunehmenden Einsatzes (11).

12. Gelenkkugelaufnahme einer Schultergelenkprothese mit einem Einsatz (11) nach einem der Ansprüche 1 bis 9 und einer Schale (13) nach einem der Ansprüche 10 oder 11.

13. Schultergelenkprothese mit einer Gelenkkugelaufnahme (11, 13) nach Anspruch 12.

14. Schultergelenkprothese nach Anspruch 13,
**dadurch gekennzeichnet,**
**dass** die Gelenkkugelaufnahme (11, 13) zur Befestigung am Humerus vorgesehen ist.

## Claims

1. An insert of a joint ball mount of a shoulder joint prosthesis,
wherein the insert (11) has a first side (I) having a recess (15) for the reception of a joint ball and a second side (II) which is provided and formed for mounting in a shell (13) of the joint ball mount; wherein a snap-action mechanism (19) extending around a center axis (17) of the insert (11) is arranged in the region of the second side (II) and, viewed from the second side (II), has, together with a collar (21), an undercut (23) extending around the center axis (17) such that the collar (21) can be brought by elastic deformation into undercut engagement with a correspondingly formed counter-element (25) of the shell (13) and such that the snap-action mechanism (19) is interrupted at at least one point (31) in the peripheral direction of the insert (11); and
wherein the insert (11) has a cylindrical guide region (27) at the outer periphery in the region provided for the mounting in the shell (13), with the outer diameter of said cylindrical guide region being larger than the maximum outer diameter of the snap-action mechanism and said cylindrical guide region being arranged toward the first side, viewed from the snap-action mechanism, **characterized in that** the insert has a second guide region at the second side which is arranged toward the second side in the axial direction of the insert, viewed from the snap-action mechanism.

2. An insert in accordance with claim 1,
**characterized in that**
the axis of the cylindrical guide region (27) coincides with the center axis (17) of the insert (11).

3. An insert in accordance with one of the claims 1 or 2,
**characterized in that** the insert (11) has a maximum outer diameter in the region of the first side (I) which is adjoined by the cylindrical guide region (27) after a step-like diameter reduction.

4. An insert in accordance with any one of the preceding claims,
**characterized in that**
the cylindrical guide region has means (37, 235) for the shape-matched security against rotation of the insert.

5. An insert in accordance with any one of the preceding claims,
**characterized in that**
the outer diameter of the cylindrical guide region (27) represents the largest diameter of the insert (11) disposed within the shell (13) in the state coupled to the shell (13).

6. An insert in accordance with any one of the preceding claims,
**characterized in that**
a respective ring-shaped surface (47, 49), of which one is formed as an axial abutment surface for the shell (13), adjoins the two axial ends of the cylindrical guide region (27) and extends in each case transversely, in particular perpendicular, to the axis of the cylindrical guide region (27).

7. An insert in accordance with any one of the preceding claims,
**characterized in that**
the recess (15) has a spherical surface region (51) which extends over an angular range (Ω) of less than 120° with respect to a center (M) of the spherical surface region (51).

8. An insert in accordance with any one of the preceding claims,
**characterized in that**
the axial end of the cylindrical guide region (27) facing the first side (I) adopts an axial position at which a tangent at a spherical surface region (51) of the recess (15) includes a gradient angle (ω) of less than 60°, in particular of less than 45°, with a perpendicular to the center axis (17).

9. An insert in accordance with any one of the preceding claims,
**characterized in that**
the cylindrical guide region (27) adopts an axial position in the direction of the center axis (17) which is located in the region of the axial position of a pole of the recess (15).

10. A shell of a joint ball mount of a shoulder joint prosthesis for the mounting of an insert in accordance with any one of the preceding claims,
**characterized in that**
the shell (13) has a cylindrical inner guide region (33) at its inner side for the mounting of the cylindrical guide region (27) of the insert (11) and furthermore has a further inner guide region at the base of the mount opening.

11. A shell in accordance with claim 10,
**characterized in that**
the axial extent of the cylindrical inner guide region (33) of the shell (13) is at least as large and in particular substantially precisely as large as the axial extent (x) of the cylindrical guide region (27) of the insert (11) to be mounted.

12. A joint ball mount of a shoulder joint prosthesis having an insert (11) in accordance with any one of the claims 1 to 9 and having a shell (13) in accordance with one of the claims 10 or 11.

13. A shoulder joint prosthesis having a joint ball mount (11, 13) in accordance with claim 12.

14. A shoulder joint prosthesis in accordance with claim 13,
**characterized in that**
the joint ball mount (11, 13) is provided for the fastening to the humerus.

## Revendications

1. Insert d'un moyen de réception de rotule d'une prothèse de l'articulation de l'épaule, l'insert (11) comprenant un premier côté (I) pourvu d'un renfoncement (15) pour recevoir une rotule ainsi qu'un second côté (II) prévu et réalisé pour la réception dans une coque (13) du moyen de réception de rotule, dans lequel
dans la zone du second côté (II), il est prévu un mécanisme d'encliquetage (19) qui s'étend autour d'un axe central (17) de l'insert (11) et qui, vu depuis le second côté (II), présente conjointement avec une collerette (21) une contre-dépouille (23) s'étendant autour de l'axe central (17) de telle sorte que par déformation élastique la collerette (21) est susceptible d'être amenée en engagement par l'arrière avec un élément antagoniste correspondant (25) de la coque (13), et que le mécanisme d'encliquetage (19) est interrompu à au moins un emplacement (31) en direction périphérique de l'insert (11), et
dans la zone prévue pour la réception dans la coque (13), l'insert (11) présente une zone de guidage cylindrique (27) à la périphérie extérieure, dont le diamètre extérieur est supérieur au diamètre extérieur maximal du mécanisme d'encliquetage et qui, vue depuis le mécanisme d'encliquetage, est agencée vers le premier côté,
**caractérisé en ce que** sur le second côté, l'insert présente une seconde zone de guidage qui, vue depuis le mécanisme d'encliquetage en direction axiale de l'insert, est agencée vers le second côté.

2. Insert selon la revendication 1,
**caractérisé en ce que**
l'axe de la zone de guidage cylindrique (27) coïncide avec l'axe central (17) de l'insert (11).

3. Insert selon l'une des revendications 1 ou 2,
**caractérisé en ce que**
dans la zone du premier côté (I), l'insert (11) présente un diamètre extérieur maximal auquel se raccorde la zone de guidage cylindrique (27) après une réduction du diamètre en forme de gradin.

4. Insert selon l'une des revendications précédentes,
**caractérisé en ce que**
la zone de guidage cylindrique comprend des moyens (37, 235) destinés au blocage anti-rotation par coopération de formes de l'insert.

5. Insert selon l'une des revendications précédentes,
**caractérisé en ce que**
dans l'état couplé à la coque (13), le diamètre extérieur de la zone de guidage cylindrique (27) représente le diamètre de l'insert (11) le plus grand à l'intérieur de la coque (13).

6. Insert selon l'une des revendications précédentes,
**caractérisé en ce que**
aux deux extrémités axiales de la zone de guidage cylindrique (27) se raccorde respectivement une zone annulaire (47, 49) qui s'étend transversalement, en particulier perpendiculairement à l'axe de la zone de guidage cylindrique (27), dont l'une est réalisée sous forme de surface de butée axiale pour la coque (13).

7. Insert selon l'une des revendications précédentes,
**caractérisé en ce que**
le renfoncement (15) présente une zone de surface sphérique (51) qui s'étend sur une zone angulaire (Ω) de moins de 120° par rapport à un centre (M) de la zone de surface sphérique (51).

8. Insert selon l'une des revendications précédentes,
**caractérisé en ce que**
l'extrémité axiale de la zone de guidage cylindrique (27), tournée vers le premier côté (I), occupe une position axiale dans laquelle une tangente à une zone de surface sphérique (51) du renfoncement (15) définit un angle d'inclinaison (ω) de moins de 60°, en particulier de moins de 45°, avec une perpendiculaire à l'axe central (17).

9. Insert selon l'une des revendications précédentes,
**caractérisé en ce que**
en direction de l'axe central (17), la zone de guidage cylindrique (27) occupe une position axiale qui se trouve au niveau de la position axiale d'un pôle du renfoncement (15).

10. Coque d'un moyen de réception de rotule d'une prothèse de l'articulation de l'épaule pour la réception d'un insert selon l'une des revendications précédentes,
**caractérisée en ce que**
la coque (13) présente sur con côté intérieur une zone de guidage cylindrique intérieure (33) pour recevoir la zone de guidage cylindrique (27) de l'insert (11) et présente en outre une autre zone de guidage intérieure au fond de l'ouverture de réception.

11. Coque selon la revendication 10,
**caractérisée en ce que**
l'extension axiale de la zone de guidage cylindrique intérieure (33) de la coque (13) est au moins et en particulier sensiblement aussi grande que l'extension axiale (x) de la zone de guidage cylindrique (27) de l'insert (11) à recevoir.

12. Moyen de réception de rotule d'une prothèse de l'articulation de l'épaule comportant un insert (11) selon l'une des revendications 1 à 9 et une coque (13) selon l'une des revendications 10 ou 11.

13. Prothèse de l'articulation de l'épaule comportant un moyen de réception de rotule (11, 13) selon la revendication 12.

14. Prothèse de l'articulation de l'épaule selon la revendication 13, **caractérisée en ce que**
le moyen de réception de rotule (11, 13) est prévu pour la fixation sur l'humérus.
